# FASCICULE DE BREVET EUROPEEN

(11) **EP 1 217 002 B1**
(45) Date de publication et mention de la délivrance du brevet: **22.09.2004**
(21) Numéro de dépôt: 01403333.6
(22) Date de dépôt: 21.12.2001
(51) Int. Cl.: C07D 491/04, C07D 417/14, C07D 405/14, A61K 31/4433, A61P 19/02

(54) **2-(4-pyridinyl)-benzofurans comme inhibiteurs de métalloprotéases**
2-(4-Pyridinyl)-Benzofurane zur Verwendung als Metalloprotease-Inhibitoren
2-(4-pyridinyl)-benzofurans as metalloprotease inhibitors

(30) Priorité: 22.12.2000 FR 0016826
(43) Date de publication de la demande: 26.06.2002
(73) Titulaire: Les Laboratoires Servier, 92200 Neuilly-sur-Seine (FR)
(72) Inventeur: De Nanteuil, Guillaume, 92150 Suresnes (FR); Benoist, Alain, 95130 Franconville (FR); Pastoureau, Philippe, 92310 Sèvres (FR); Sabatini, Massimo, 92380 Garches (FR); Hickman, John, 75017 Paris (FR); Pierre, Alain, 78580 Les Alluets Le Roi (FR); Tucker, Gordon, 75017 Prais (FR)

(56) Documents cités:
- US-A- 5 866 587

## Description

La présente invention concerne de nouveaux inhibiteurs de métalloprotéases, leur procédé de préparation et les compositions pharmaceutiques qui les contiennent.

A l'état physiologique, la synthèse des tissus connectifs est en équilibre dynamique avec la dégradation de la matrice extracellulaire. Cette dégradation est due à des protéases à zinc (métalloprotéases) secrétées par les cellules de la matrice existante : ce sont de façon non limitative les collagénases (MMP-1, MMP-8, MMP-13), les gélatinases ou collagénases de type IV (MMP-2, MMP-9) et les stromélysines (MMP-3).

A l'état normal, ces enzymes cataboliques sont régulées au niveau de leur synthèse et de leur sécrétion, ainsi qu'au niveau de leur activité enzymatique extracellulaire par des inhibiteurs naturels, comme l'α₂-macroglobuline ou les TIMP (Tissue Inhibitor of MetalloProteinase) qui forment des complexes inactifs avec les métalloprotéases.

Le point commun aux pathologies impliquant ces enzymes est un déséquilibre entre l'activité des enzymes activées et celle de leurs inhibiteurs naturels, avec pour conséquence une dégradation excessive des tissus.

La dégradation incontrôlée et accélérée des membranes par la résorption de la matrice extracellulaire catalysée par les métalloprotéases est un paramètre commun à plusieurs conditions pathologiques comme l'arthrite rhumatoïde, l'arthrose, l'invasion et la croissance tumorale, y compris la dissémination maligne et la formation de métastases, les ulcérations, l'athérosclérose, etc...

Récemment, le BB94, inhibiteur de métalloprotéases a montré une activité antitumorale en clinique où il s'est révélé actif sur les cancers de l'ovaire (Becket et al., DDT 1996, 1 (1), 16).

On peut donc attendre d'un inhibiteur de métalloprotéases qu'il restaure l'équilibre entre protéase et inhibiteur et de ce fait modifie de façon favorable l'évolution de ces pathologies.

Un certain nombre d'inhibiteurs de métalloprotéases ont été décrits dans la littérature. C'est le cas, plus particulièrement, des composés décrits dans les brevets WO 95/35275, WO 95/35276, EP 606 046, WO 96/00214, EP 803 505, US 5,866,587 WO 97/20824 ou EP 780 386.

Les composés de la présente invention, outre le fait qu'ils soient nouveaux, se sont révélés être des inhibiteurs de métalloprotéases plus puissants que ceux décrits dans la littérature ce qui les rend donc potentiellement utiles pour le traitement des cancers, des maladies rhumatismales comme l'arthrose et l'arthrite rhumatoïde, de l'athérosclérose, etc...

Plus spécifiquement, la présente invention concerne les composés de formule (I): 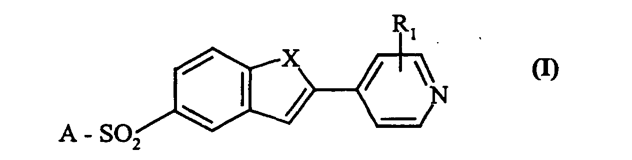 dans laquelle :
- R₁: représente un atome d'hydrogène, d'halogène, un groupement alkyle (C₁-C₆) linéaire ou ramifié ou un groupement alkoxy (C₁-C₆) linéaire ou ramifié,
- X: représente un atome d'oxygène, de soufre ou un groupement NR dans lequel R représente un atome d'hydrogène ou un groupement alkyle (C₁-C₆) linéaire ou ramifié,
- A: représente l'un quelconque des groupements suivants :
● 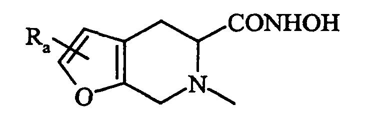 dans lequel Rₐ représente un atome d'hydrogène, d'halogène, un groupement alkyle (C₁-C₆) linéaire ou ramifié ou alkoxy (C₁-C₆) linéaire ou ramifié,
● 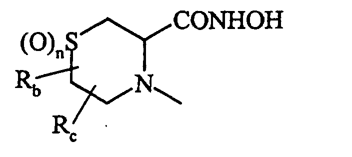 dans lequel R_{b}, R_{c}, identiques ou différents, représentent un atome d'hydrogène ou un groupement alkyle (C₁-C₆) linéaire ou ramifié, n représente 0, 1 ou 2,
● ou, 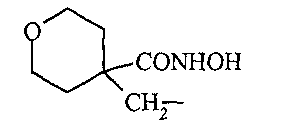
leurs isomères, N-oxydes, ainsi que leurs sels d'addition à un acide ou à une base pharmaceutiquement acceptable.

Parmi les acides pharmaceutiquement acceptables, on peut citer à titre non limitatif les acides chlorhydrique, bromhydrique, sulfurique, phosphonique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, l'hydroxyde de lithium, la triéthylamine, la tertbutylamine, etc...

Les composés préférés de l'invention sont les composés de formule (I) dans laquelle X représente un atome d'oxygène.

R₁ est préférentiellement un atome d'hydrogène.

Lorsque A représente un groupement 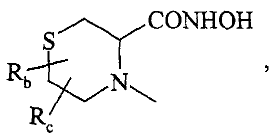 celui-ci est préférentiellement le groupement

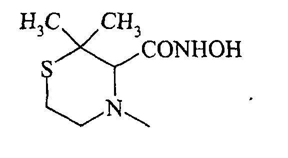

Les composés préférés de l'invention sont :
- le N-hydroxy-(5R)-6-{[2-(4-pyridinyl)-1-benzofuran-5-yl]sulfonyl}-4,5,6,7-tétrahydrofuro[2,3-c]pyridine-5-carboxamide,
- le N-hydroxy-(3S)-2,2-diméthyl-4-{[2-(4-pyridinyl)-1-benzofuran-5-yl]sulfonyl}-3-thiomorpholinecarboxamide,
- le N-hydroxy-4-{{[2-(4-pyridinyl)-1-benzofuran-5-yl]sulfonyl}méthyl}tétrahydro-2*H*-pyran-4-carboxamide,
ainsi que leurs sels d'addition.

L'invention s'étend également au procédé de préparation des composés de formule (I).

Lorsque les composés de formule (I) sont tels que A représente l'un quelconque des groupements : 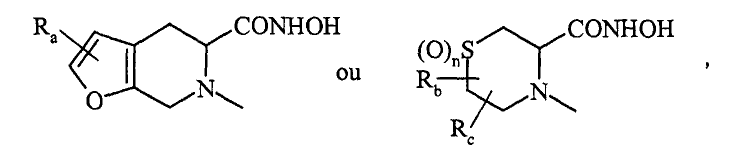 ce procédé est caractérisé en ce que l'on utilise comme produit de départ un composé de formule (II) : 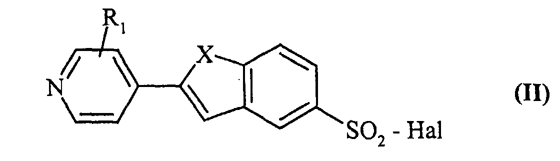 dans laquelle R₁ et X ont la même signification que dans la formule (I) et Hal représente un atome d'halogène, que l'on fait réagir sur l'un quelconque des composés (IIIa) ou (IIIb), sous forme racémique ou d'isomère défini : 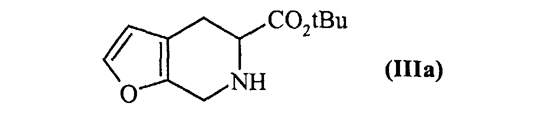 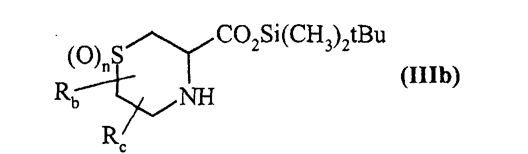 dans laquelle Rb, Rc et n ont la même signification que dans la formule (I),
pour conduire respectivement aux composés de formule (IVa) ou (IVb): 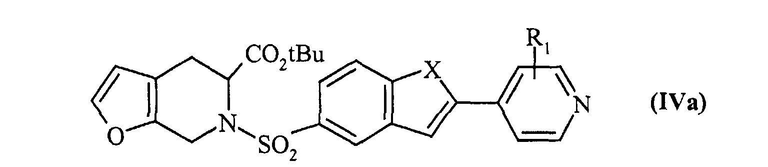 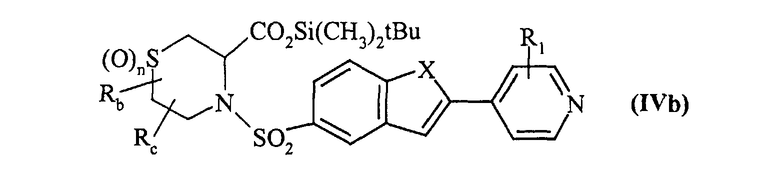 dans lesquelles X, R₁, Rb, Rc et n ont la même signification que dans la formule (I),
que l'on déprotège pour conduire respectivement aux composés de formule (Va) ou (Vb), 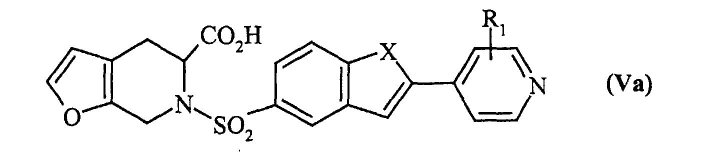 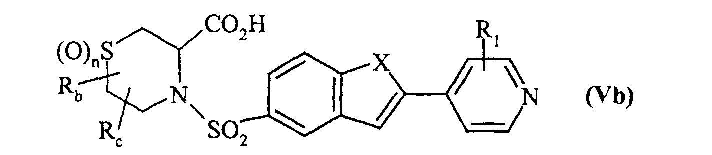 dans lesquelles X, R₁, Rb, Rc et n ont la même signification que dans la formule (I),
que l'on soumet à l'action d'une hydroxylamine-O-substituée, pour conduire, après déprotection de la fonction hydroxamate, respectivement aux composés de formule (I/a) ou (I/b) : 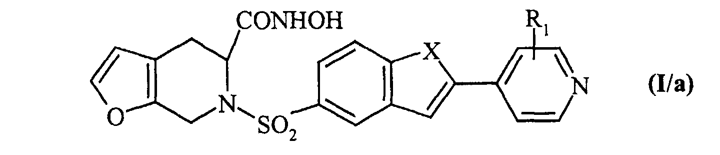 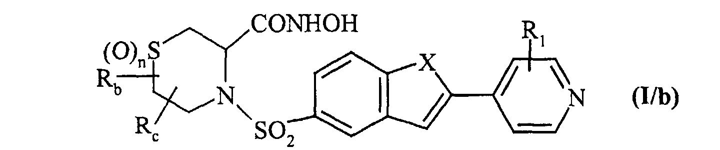 dans lesquelles X, R₁, Rb, Rc et n ont la même signification que dans la formule (I),
composés de formule (I/a) ou (I/b) :
- que l'on transforme éventuellement en N-oxydes correspondants,
- que l'on purifie le cas échéant selon une technique classique de purification,
- dont on sépare éventuellement les isomères selon une technique classique de séparation et
- que l'on transforme, si on le souhaite, en leurs sels d'addition à un acide ou à une base pharmaceutiquement acceptable.

Lorsque les composés de formule (I) sont tels que A représente le groupement : 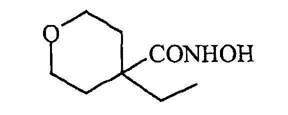 le procédé est caractérisé en ce que l'on utilise comme produit de départ un composé de formule (VI) : 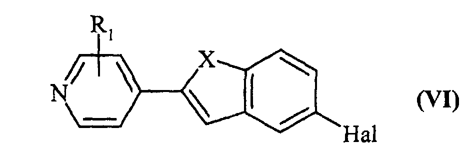 dans laquelle R₁ et X ont la même signification que dans la formule (I), et Hal représente un atome d'halogène,
que l'on fait réagir sur un composé de formule (VII) : 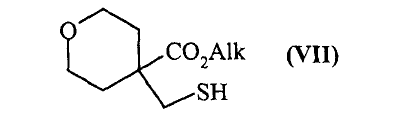 dans laquelle Alk représente un groupement alkyle (C₁-C₆) linéaire ou ramifié, pour conduire après hydrolyse acide au composé de formule (VIII) : 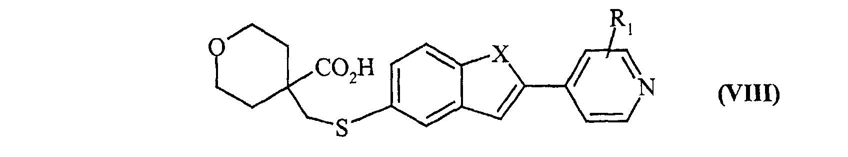 dans laquelle R₁ et X ont la même signification que dans la formule (I),
sur lequel on fait réagir un réactif d'oxydation,
pour conduire au composé de formule (IX) : 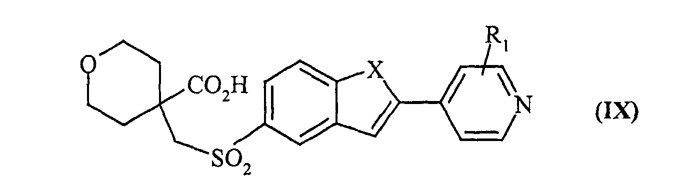 dans laquelle X et R₁ ont la même signification que dans la formule (I),
que l'on soumet à l'action d'une hydroxylamine-O-substituée, pour conduire après déprotection de la fonction hydroxamate au composé de formule (I/c), cas particulier des composés de formule (I): 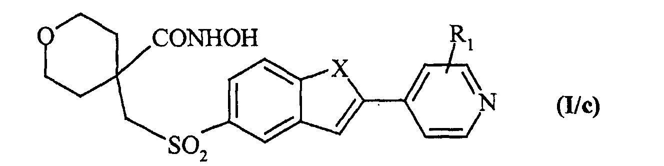 dans laquelle R₁ et X ont la même signification que dans la formule (I),
que l'on transforme éventuellement en N-oxydes correspondants,
que l'on purifie le cas échéant selon une technique classique de purification, dont on sépare éventuellement les isomères selon une technique classique de séparation et dont on transforme, si on le souhaite, en ses sels d'addition à un acide ou à une base pharmaceutiquement acceptable.

Les composés de formule (II) et (VI) sont obtenus en utilisant comme produit de départ un composé de formule (X) : 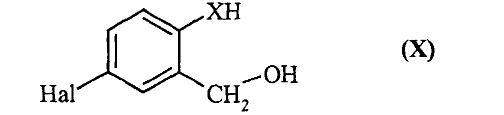 dans laquelle Hal représente un atome d'halogène et X a la même signification que dans la formule (I),
que l'on fait réagir avec le bromure de triphénylphosphine pour conduire au composé de formule (XI) : 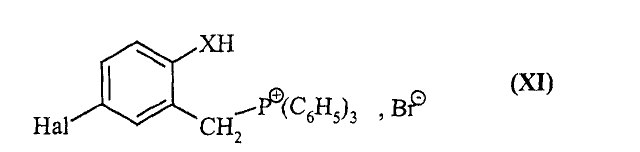 dans laquelle Hal et X ont la même signification que précédemment,
que l'on fait réagir avec un chlorure d'isonicotinoyle de formule (XII) : 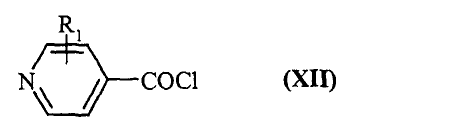 dans laquelle R₁ a la même signification que dans la formule (I),
pour conduire au composé de formule (VI) : 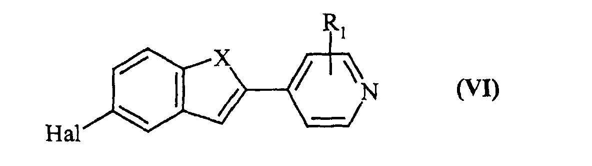 dans laquelle Hal, X et R₁ ont la même signification que précédemment,
qui est ensuite transformé en composé de formule (XIII) en présence d'oxyde de soufre et de n-butyllithium : 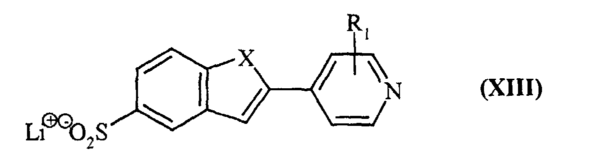 dans laquelle R₁ et X ont la même signification que précédemment,
puis en composé de formule (II) en présence d'halogènure de sulfuryle : 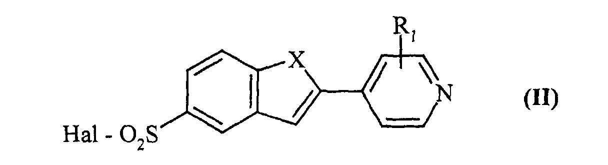 dans laquelle Hal, X et R₁ ont la même signification que précédemment.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 0,01 à 2 g par jour en une ou plusieurs prises.

Les exemples suivants illustrent l'invention mais ne la limitent en aucune façon.

Les produits de départ utilisés sont des produits connus ou préparés selon des modes opératoires connus.

Les préparations conduisent à des intermédiaires de synthèse, utiles pour la préparation des composés de l'invention.

Les structures des composés décrits dans les exemples et les préparations ont été déterminées selon les techniques spectrophotométriques usuelles (infrarouge, RMN, spectrométrie de masse, ...).

### PREPARATION A : 4-(Sulfanylméthyl)tétrahydro-2H-pyran-4-carboxylate d'éthyle

### STADE A : 4-[(Acétylsulfanyl)méthyl]tétrahydro-2H-pyran-4-carboxylate d'éthyle

Sous argon, 47 g de triphénylphosphine sont dissous dans 350 ml de tétrahydrofurane (THF). Après refroidissement à 0°C, 34,9 ml d'azodicarboxylate de diisopropyle (DIAD) sont ajoutés à cette solution. Après 30 minutes d'agitation, on ajoute une solution contenant 89 mmoles de 4-(hydroxyméthyl)tétrahydro-2*H*-pyran-4-carboxylate d'éthyle et 12,8 ml d'acide thioacétique dans 300 ml de THF.
Après une nuit d'agitation à température ambiante, et évaporation à sec, le résidu est repris par de l'éther. Après filtration, le filtrat est évaporé et conduit au produit attendu sous forme d'huile qui est purifiée par chromatographie sur colonne de silice en utilisant comme éluant un mélange dichlorométhane/acétate d'éthyle (90/10).

### STADE B : 4-(Sulfanylméthyl)tétrahydro-2H-pyran-4-carboxylate d'éthyle

A 67,4 mmoles du composé obtenu au stade précédent dissoutes dans 50 ml d'éthanol sont ajoutés 92 ml d'une solution d'acide chlorhydrique 2,2N dans l'éthanol.
Après une nuit d'agitation, l'ensemble est évaporé à sec pour conduire au produit attendu sous forme d'huile.

### PREPARATION B : 5-Bromo-2-(4-pyridinyl)benzofurane

### STADE A : Bromure de (5-bromo-2-hydroxybenzyl)triphénylphosphonium

A 490 mmoles de 4-bromo-2-(hydroxyméthyl)phénol en suspension dans 500 ml d'acétonitrile sont ajoutés 169 g de bromure de triphénylphosphine. L'ensemble est chauffé à 100°C pendant 2 heures. Après refroidissement, le précipité formé est filtré, séché et conduit au produit attendu.
*Point de fusion : 260°C*

### STADE B : 5-Bromo-2-(4-pyridinyl)benzofurane

A 243 g du produit obtenu au stade précédent placés dans 2 litres de toluène en présence de 90,1 g de chlorure d'isonicotinoyle sont ajoutés 256 ml de triéthylamine. L'ensemble est chauffé 24 heures à 100°C. Après refroidissement, le précipité formé est filtré et conduit au produit attendu après recristallisation dans de l'acétate d'éthyle.
*Point de fusion : 160°C*

### PREPARATION C : Chlorure de 2-(4-pyridinyl)-1-benzofuran-5-sulfonyle

### STADE A : {[2-(4-Pyridinyl)-1-benzofuran-5-yl]sulfonyl}lithium

A 60,2 mmoles du composé obtenu dans la préparation B en suspension dans du tétrahydrofurane, sont ajoutés à -72°C du n-butyllithium. Après 90 minutes à -72°C, on fait passer un courant de SO₂ pendant 1 heure. Après 2 jours à température ambiante, le solide formé est filtré, rincé à l'éther et conduit au produit attendu.

### STADE B : Chlorure de 2-(4-pyridinyl)-1-benzofuran-5-sulfonyle

59 mmoles du produit obtenu au stade précédent sont mises en suspension dans 80 ml de dichlorométhane. Après refroidissement à 0°C, 5,7 ml de chlorure de sulfuryle sont ajoutés goutte à goutte. Après une nuit à température ambiante, le précipité formé est filtré et rincé à l'éther et conduit au produit attendu.
*Point de fusion : 210°C*

### EXEMPLE 1 : N-Hydroxy-(5R)-6-{[2-(4-pyridinyl)-1-benzofuran-5-yl]sulfonyl}-4,5,6,7-tétrahydrofuro[2,3-c]pyridine-5-carboxamide, chlorhydrate

### STADE A : t-Butyl ester de l'acide (5R)-6-{[2-(4-pyridinyl)-1-benzofuran-5-yl]sulfonyl}-4,5,6,7-tétrahydrofuro[2,3-c]pyridine-5-carboxylique

30 mmoles de chlorhydrate du *t*-butyl ester de l'acide (5R)-4,5,6,7-tétrahydrofuro[2,3-c]pyridine-5-carboxylique sont placées dans 150 ml de pyridine. 30 mmoles de chlorure de 2-(4-pyridinyl)-1-benzofuran-5-sulfonyle décrit dans la préparation C sont alors ajoutées à température ambiante et l'ensemble est chauffé à 60°C une nuit.
Après évaporation de la pyridine, reprise du résidu par du dichlorométhane, lavage à l'eau, séchage et évaporation on obtient le produit attendu sous forme d'une huile qui est purifiée par chromatographie sur silice en utilisant comme éluant un mélange dichlorométhane/éthanol (98/2).

### STADE B : Acide-(5R)-6-{[2-(4-pyridinyl)-1-(benzofuran-5-yl]sulfonyl}-4,5,6,7-tétrahydrofuro[2,3-c]pyridine-5-carboxylique

A 22 mmoles de l'ester obtenu au stade précédent dissoutes dans 250 ml de dichlorométhane sont ajoutés 2,4 ml d'anisole. L'ensemble est refroidi à 0°C et 17 ml d'acide trifluoroacétique sont additionnés. L'ensemble est maintenu une nuit à température ambiante. Après évaporation à sec, le résidu est purifié par chromatographie sur silice en utilisant comme éluant un mélange dichlorométhane/méthanol (85/15) et conduit au produit attendu.

### STADE C : N-Allyloxy-(5R)-6-{[2-(4-pyridinyl)-1-benzofuran-5-yl]sulfonyl}-4,5,6,7-tétrahydrofuro[2,3-c]pyridine-5-carboxamide

A une solution refroidie à 0°C, contenant 12 mmoles de l'acide obtenu au stade précédent dans 150 ml de dichlorométhane, sont ajoutés 10,3 ml de diisopropyléthylamine, 1,65 g de 1-hydroxybenzotriazole, une solution contenant 1,6 g de chlorhydrate de 0-allylhydroxylamine dans 50 ml de diméthylformamide, et 4,65 g de tétrafluoroborate de O-benzotriazolyl-tétraméthylisouronium (TBTU). L'ensemble est maintenu une nuit à température ambiante. Après évaporation à sec, le résidu est repris par du dichlorométhane. Après lavage à l'eau, séchage et évaporation, on obtient un résidu qui conduit au produit attendu après purification sur colonne de silice en utilisant comme éluant un mélange dichlorométhane/éthanol/ammoniaque (98/2/0,2).

### STADE D : N-Hydroxy-(5R)-6-{[2-(4-pyridinyl)-1-benzofuran-5-yl]sulfonyl}-4,5,6,7-tétrahydrofuro[2,3-c]pyridine-5-carboxamide, chlorhydrate

A 8,55 mmoles du produit obtenu au stade précédent dans 150 ml de dichlorométhane, on ajoute 300 mg du catalyseur Pd (PPh₃)₂ Cl₂ et 1,5 ml d'acide acétique. Après 5 minutes, 4,9 ml d'hydrure de tributylétain sont ajoutés. L'ensemble est maintenu 30 minutes à température ambiante puis évaporé. Après reprise du résidu par de l'acétonitrile, 20 ml d'acide chlorhydrique 1N sont ajoutés et l'ensemble est dilué par de l'eau. La phase aqueuse est lavée à l'éther puis lyophilisée et conduit au produit attendu.

| *Microanalyse élémentaire :* | | | | | |
|---|---|---|---|---|---|
| | *C%* | *H%* | *N%* | *Cl%* | *S%* |
| *calculé* | *53,00* | *3,81* | *8,83* | *7,45* | *6,74* |
| *trouvé* | *52,94* | *3,81* | *8,70* | *7,30* | *6,65* |

### EXEMPLE 2 : N-Hydroxy-(3S)-2,2-diméthyl-4-{[2-(4-pyridinyl)-1-benzofuran-5-yl]sulfonyl}-3-thiomorpholinecarboxamide

### STADE A : tert-Butyl(diméthyl)silyl ester de l'acide 4-{[2-(4-aminophényl)-1-benzofuran-5-yl]sulfonyl}-2,2-diméthyl-3-thiomorpholine carboxylique

58,7 mmoles de *tert*-butyl(diméthyl)silyl ester de l'acide 2,2-diméthyl-3-thiomorpholine carboxylique sont dissoutes dans 500 ml de dichlorométhane anhydre. A -20°C, on ajoute 16 ml de N-méthyl morpholine, puis 57,5 mmoles de chlorure de 2-(4-pyridinyl)-1-benzofuran-5-sulfonyle décrit dans la préparation C. L'ensemble est agité 48 heures à température ambiante puis versé sur 300 ml d'eau. Après décantation, lavage à l'eau, séchage et évaporation, on obtient le produit attendu sous forme d'huile.

### STADE B : Acide 4-{[2-(4-aminophényl)-1-benzofuran-5-yl}-2,2-diméthyl-3-thiomorpholine carboxylique

33 g du composé obtenu au stade précédent sont dissous dans 400 ml de méthanol anhydre. L'ensemble est porté 2 heures à reflux puis évaporé. Le produit attendu est obtenu après cristallisation du résidu dans de l'éther.

### STADE C : N-(Allyloxy)-4-{[2-(4-aminophényl)-1-benzofuran-5-yl]sulfonyl}-2,2-diméthyl-3-thiomorpholine carboxamide

Le produit attendu est obtenu selon le procédé décrit au stade C de l'exemple 1 à partir du produit décrit au stade précédent.

### STADE D : N-Hydroxy-(3S)-2,2-diméthyl-4-{[2-(4-pyridinyl)-1-benzofuran-5-yl] sulfonyl}-3-thiomorpholinecarboxamide

A 10,2 mmoles du composé décrit au stade précédent en solution dans 70 ml de dichlorométhane, on ajoute 360 mg du catalyseur Pd (PPh₃)₂ Cl₂ et 1,75 ml d'acide acétique puis après 5 minutes 5,8 ml d'hydrure de tributylétain. Après 20 minutes d'agitation, 70 ml d'éther sont ajoutés. L'insoluble est filtré et lavé à l'éther. Il est repris par un mélange acétonitrile/eau (50/50) et conduit au produit attendu après lyophilisation.

| *Microanalyse élémentaire :* | | | | |
|---|---|---|---|---|
| | *C%* | *H%* | *N%* | *S%* |
| *calculé* | *53,68* | *4,73* | *9,39* | *14,33* |
| *trouvé* | *53,38* | *4,81* | *9,08* | *13,91* |

### EXEMPLE 3 : N-Hydroxy-4-{{[2-(4-pyridinyl)-1-benzofuran-5-yl]sulfonyl} méthyl}tétrahydro-2H-pyran-4-carboxamide

### STADE A : Ester éthylique de l'acide 4-{{[2-(4-pyridinyl)-1-benzofuran-5-yl] sulfanyl}méthyl}tétrahydro-2H-pyran-4-carboxylique

Dans 75 ml de N-méthylpyrrolidone sont placés 23,9 mmoles de 5-bromo-2-(4-pyridinyl)benzofurane décrit dans la préparation B, 7,32 g du composé décrit dans la préparation A, 431 mg de tris-(dibenzylidèneacétone)dipalladium et 1,05 g de 1,1'-bis(diphényl)phosphinoferrocène. L'ensemble est chauffé à 100°C pendant 48 heures. Après évaporation, le résidu est repris par de l'acétate d'éthyle et une solution saturée de chlorure de sodium. Après filtration, extraction à l'acétate d'éthyle et évaporation, le résidu est purifié par chromatographie sur colonne de silice en utilisant comme éluant un mélange dichlorométhane/acétate d'éthyle (9/1). Le produit attendu cristallise alors.
*Point de fusion : 92°C*

### STADE B : Acide 4-{{[2-(4-pyridinyl)-1-benzofuran-5-yl]sulfanyl}méthyl}tétrahydro-2H-pyran-4-carboxylique

7,5 g de l'ester obtenu au stade précédent sont placés dans 200 ml d'acide chlorhydrique 6N. L'ensemble est porté au reflux une nuit. Après refroidissement, le pH de la solution est ajusté à 7 par addition de soude. Le précipité qui se forme est filtré et lavé à l'eau et conduit au produit attendu.
*Point de fusion : 227°C*

### STADE C : Acide 4-{{[2-(4-pyridinyl)-1-benzofuran-5-yl]sulfonyl}méthyl}tétrahydro-2H-pyran-4-carboxylique

18 mmoles du composé obtenu au stade précédent sont placés dans 116 ml d'eau et 140 ml d'acétonitrile. L'ensemble est refroidi dans un bain de glace et 17,65 g d'oxone sont ajoutés par portions. Le milieu est abandonné 48 heures à température ambiante. Après évaporation, le pH est ajusté à 7 et le produit attendu précipite.
*Point de fusion : 217°C*

### STADE D : 4-{{[2-(4-Pyridinyl)-1-benzofuran-5-yl]sulfonyl}méthyl}-N-(allyloxy)tétrahydro-2H-pyran-4-carboxamide

Le produit attendu est obtenu selon le procédé décrit au stade C de l'exemple 1 à partir du composé décrit au stade précédent.

### STADE E : N-Hydroxy-4-{{[2-(4-pyridinyl)-1-benzofuran-5-yl]sulfonyl} méthyl}tétrahydro-2H-pyran-4-carboxamide

Le produit attendu est obtenu selon le procédé décrit au stade D de l'exemple 1 à partir du composé décrit au stade précédent.

| *Microanalyse élémentaire :* | | | | |
|---|---|---|---|---|
| | *C%* | *H%* | *N%* | *S%* |
| *calculé* | *57,68* | *4,84* | *6,73* | *7,70* |
| *trouvé* | *57,71* | *4,90* | *6,19* | *7,23* |

### EXEMPLE 4 : N-Hydroxy-(3S)-2,2-diméthyl-4-{[2-(4-pyridinyl)-1-benzofuran-5-yl]sulfonyl}-3-thiomorpholinecarboxamide-1-oxyde

Le produit attendu est obtenu selon le procédé décrit dans l'exemple 2 en remplaçant au stade A l'acide 2,2-diméthyl-3-thiomorpholine carboxylique par l'acide 2,2-diméthyl-3-thiomorpholine carboxylique-1-oxyde.

### EXEMPLE 5 : N-Hydroxy-(3S)-2,2-diméthyl-4-{[2-(4-pyridinyl)-1-benzofuran-5-yl]sulfonyl}-3-thiomorpholinecarboxamide-1,1-dioxyde

Le produit attendu est obtenu selon le procédé décrit dans l'exemple 2 en remplaçant au stade A l'acide 2,2-diméthyl-3-thiomorpholine carboxylique par l'acide 2,2-diméthyl-3-thiomorpholine carboxylique-1,1-dioxyde.

### EXEMPLE 6 : N-Hydroxy-(3S)-2,2-diméthyl-4-{[2-(4-pyridinyloxyde)-1-benzofuran-5-yl]sulfonyl}-3-thiomorpholinecarboxamide

Le produit attendu est obtenu par oxydation du composé décrit dans l'exemple 2.

### ETUDE PHARMACOLOGIQUE DES DERIVES DE L'INVENTION

### EXEMPLE A : Inhibition enzymatique des métalloprotéases

Les six enzymes humaines recombinantes -MMP-1 (collagénase interstitielle), MMP-2 (gélatinase A), MMP-3 (stromélysine 1), MMP-8 (collagénase des neutrophiles), MMP-9 (gélatinase B) et MMP-13 (collagénase 3)- sont activées à l'APMA (acétate 4-aminophénylmercurique).
Les tests enzymatiques des MMP-1, -2, -8, -9 et -13 sont effectués avec le substrat peptido mimétique suivant : DnpProGhaGlyCys(Me)HisAlaLys(Nma)NH2, clivé entre la Glycine et la Cystéine pour produire un dérivé fluorescent décrit par D.M. BICKETT et coll. (Anal. Biochem., 212, 58-64, 1993).

Le test enzymatique de la MMP-3 est effectué avec le substrat peptido mimétique suivant : McaArgProLysProTyrAlaNvaTrpMetLys(Dnp)NH2, clivé entre Alanine et Norvaline pour produite un dérivé fluorescent décrit par H. NAGASE et coll. (J. Biol. Chem., 269, 20952-20957, 1994).
Les réactions conduites dans un tampon 50 mM Tris, 200 mM NaCl, 5 mM CaCl₂, 0,1 % Brij 35 à pH 7.7 sont initiées avec 20 µM de substrat dans un volume total de 100 µl à 37°C.
La fluorescence obtenue après six heures est lue en plaque 96-puits dans un fluorimètre équipé avec une combinaison de filtres de 360 nm et 460 nm pour l'excitation et l'émission. Les composés de l'invention ont des IC₅₀ pour l'ensemble des MMPs à l'exception de la MMP-1, comprises entre 10⁻¹⁰ et 10⁻⁸ M. Une spécificité d'un facteur 1000 existe pour les collagénases MMP-13 et MMP-8 vis-à-vis de la collagénase MMP-1.

### EXEMPLE B : Dégradation de la matrice du cartilage in vitro

Les composés de l'invention ont été étudiés sur un modèle de destruction de la matrice du cartilage induite par l'IL-1β. Les essais, réalisés sur cartilage de lapin, concernent :
- d'une part, la dégradation du collagène : dosage colorimétrique selon la technique de Grant (GRANT R.A. Estimation of OH-proline by the autoanalyser, J. Clin. Path., 17, 685, 1964) de la fraction d'OH-proline relarguée par le tissu mis en contact pendant 2 jours avec l'IL-1β (10 ng/ml) et de la plasmine (0.1 U/ml) ;
- d'autre part, la dégradation des protéoglycanes : mesure radio-isotopique de la fraction de glycosaminoglycanes relargués par le tissu préalablement marqué au ³⁵SO₄, au cours des 24 heures de contact avec l'APMA (5x10⁻⁴M), faisant suite à 24 heures de stimulation par l'IL-1β (10 ng/ml).
Les composés de l'invention ont été étudiés par adjonction au milieu de culture pendant les 3 jours de l'essai. Pour des concentrations comprises entre 10⁻⁹ et 10⁻⁶M, ils se sont fortement opposés à la dégradation du collagène et des protéoglycanes.

### EXEMPLE C : Angiogénèse in vitro

Des tronçons d'aorte thoracique de rats mâles Fischer 344 âgées de 8 à 12 semaines sont immergés dans un gel de collagène de type I selon la méthode de Nicosia et Ottinetti (Lab. Invest., 63, 115, 1990). Après cinq jours de culture en milieu sans sérum, les préparations sont examinées au microscope et la formation de pseudo-vaisseaux quantifiée en terme de densité vasculaire après digitalisation et analyse d'image.
Pour des concentrations comprises entre 10⁻⁹ et 10⁻⁶ M, les composés de l'invention bloquent sélectivement la formation des pseudo-vaisseaux constitués de cellules endothéliales, sans affecter les cellules fibroblastiques.

### EXEMPLE D : Composition pharmaceutique

| Formule de préparation pour 1000 comprimés dosés à 100 mg | |
|---|---|
| Composé de l'exemple 1 | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 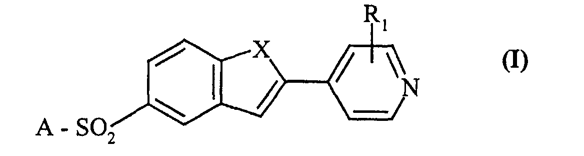 dans laquelle :
R₁ représente un atome d'hydrogène, d'halogène, un groupement alkyle (C₁-C₆) linéaire ou ramifié ou un groupement alkoxy (C₁-C₆) linéaire ou ramifié,
X représente un atome d'oxygène, de soufre ou un groupement NR dans lequel R représente un atome d'hydrogène ou un groupement alkyle (C₁-C₆) linéaire ou ramifié,
A représente l'un quelconque des groupements suivants :
● 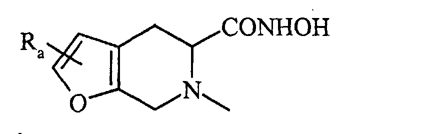 dans lequel Rₐ représente un atome d'hydrogène, d'halogène, un groupement alkyle (C₁-C₆) linéaire ou ramifié ou alkoxy (C₁-C₆) linéaire ou ramifié,
● 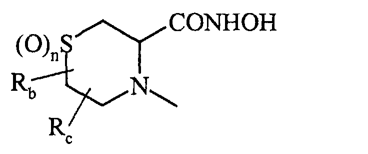 dans lequel R_{b}, R_{c}, identiques ou différents, représentent un atome d'hydrogène ou un groupement alkyle (C₁-C₆) linéaire ou ramifié, n représente 0, 1 ou 2,
● ou, 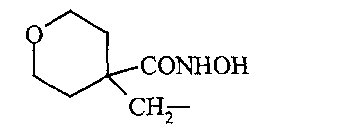
leurs isomères, N-oxydes, ainsi que leurs sels d'addition à un acide ou à une base pharmaceutiquement acceptable.

2. Composés de formule (I) selon la revendication 1 **caractérisés en ce que** A représente le groupement: 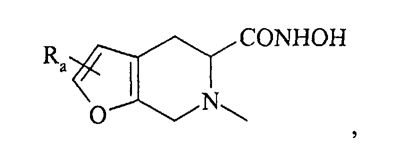 leurs isomères, ainsi que leurs sels d'addition à un acide ou à une base pharmaceutiquement acceptable.

3. Composés de formule (I) selon la revendication 1 **caractérisés en ce que** A représente le groupement : 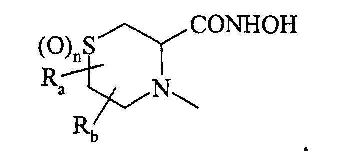 leurs isomères, ainsi que leurs sels d'addition à un acide ou à une base pharmaceutiquement acceptable.

4. Composés de formule (I) selon la revendication 1 **caractérisés en ce que** A représente le groupement : 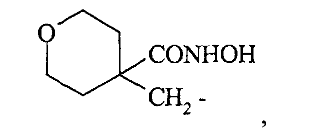 leurs isomères, ainsi que leurs sels d'addition à un acide ou à une base pharmaceutiquement acceptable.

5. Composés de formule (I) selon la revendication 3 **caractérisés en ce que** A représente le groupement : 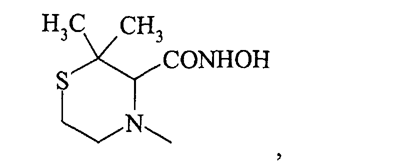 leurs isomères, ainsi que leurs sels d'addition à un acide ou à une base pharmaceutiquement acceptable.

6. Composé de formule (I) selon l'une quelconque des revendications 1 ou 2 qui est le N-hydroxy-(5R)-6-{[2-(4-pyridinyl)1-benzofuran-5-yl]sulfonyl}-4,5,6,7-tétrahydro[2,3-c] pyridine-5-carboxamide, ses isomères, ainsi que ses sels d'addition à un acide ou à une base pharmaceutiquement acceptable.

7. Composé de formule (I) selon l'une quelconque des revendications 1, 3 ou 5 qui est le N-hydroxy-(3S)-2,2-diméthyl-4-{[2-(4-pyridinyl)-1-benzofuran-5-yl]sulfonyl}-3-thiomorpholinecarboxamide, ses isomères, ainsi que ses sels d'addition à un acide ou à une base pharmaceutiquement acceptable.

8. Composé de formule (I) selon l'une quelconque des revendications 1 ou 4 qui est le N-hydroxy-4-{{[2-(4-pyridinyl)-1-benzofuran-5-yl]sulfonyl}méthyl}tétrahydro-2*H*-pyran-4-carboxamide, ses isomères, ainsi que ses sels d'addition à un acide ou à une base pharmaceutiquement acceptable.

9. Procédé de préparation des composés de formule (I) selon la revendication 1 caractérisé, lorsque les composés de formule (I) sont tels que A représente l'un quelconque des groupements : 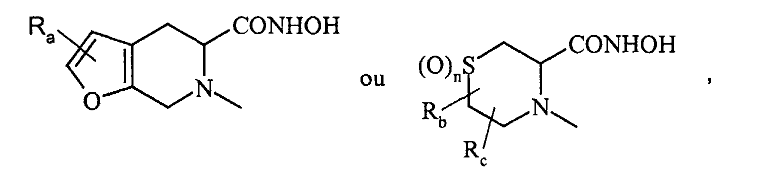 en ce que l'on utilise comme produit de départ un composé de formule (II) : 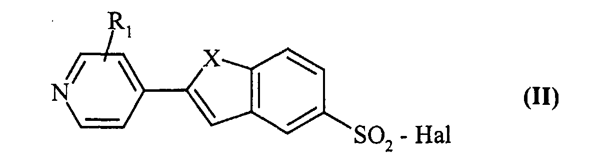 dans laquelle R₁ et X ont la même signification que dans la formule (I) et Hal représente un atome d'halogène, que l'on fait réagir sur l'un quelconque des composés (IIIa) ou (IIIb), sous forme racémique ou d'isomère défini: 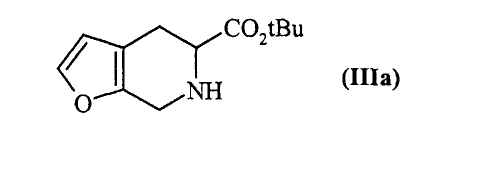 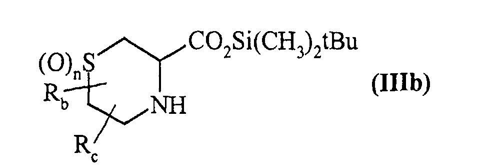 dans laquelle Rb, Rc et n sont tels que définis dans la formule (I),
pour conduire respectivement aux composés de formule (IVa) ou (IVb) : 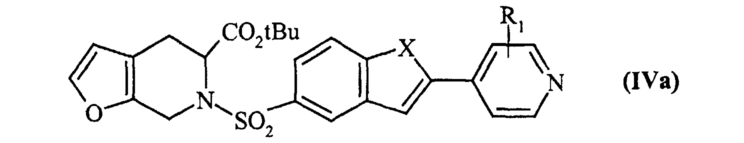 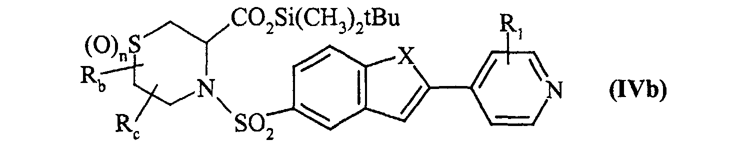 dans lesquelles X, R₁, Rb, Rc et n ont la même signification que dans la formule (I),
que l'on déprotège pour conduire respectivement aux composés de formule (Va) ou (Vb), 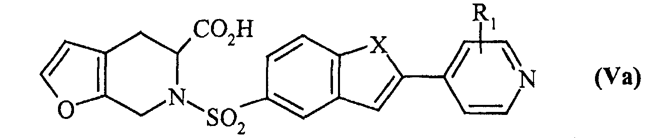 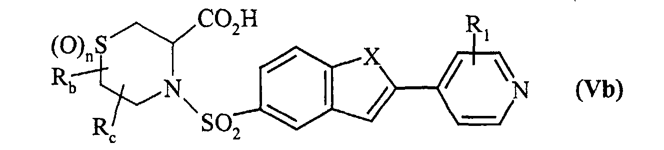 dans lesquelles X, R₁, Rb, Rc et n ont la même signification que dans la formule (I),
que l'on soumet à l'action d'une hydroxylamine-O-substituée, pour conduire, après déprotection de la fonction hydroxamate, respectivement aux composés de formule (I/a) ou (I/b) : 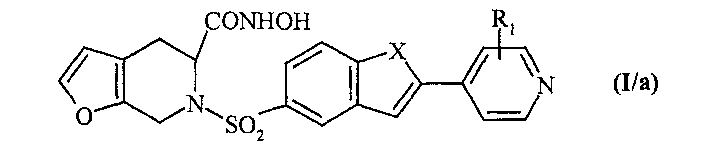 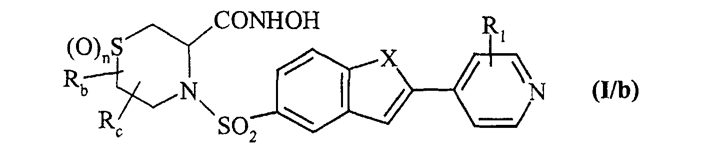 dans lesquelles X, R₁, Rb, Rc et n ont la même signification que dans la formule (I),
composés de formule (I/a) ou (I/b) :
- que l'on transforme éventuellement en N-oxydes correspondants,
- que l'on purifie le cas échéant selon une technique classique de purification,
- dont on sépare éventuellement les isomères selon une technique classique de séparation et
- que l'on transforme, si on le souhaite, en leurs sels d'addition à un acide ou à une base pharmaceutiquement acceptable.

10. Procédé de préparation des composés de formule (I) selon la revendication 1 caractérisé, lorsque les composés de formule (I) sont tels que A représente le groupement : 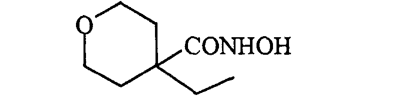 le procédé est **caractérisé en ce que** l'on utilise comme produit de départ un composé de formule (VI) : 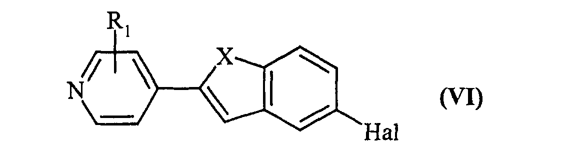 dans laquelle R₁ et X ont la même signification que dans la formule (I), et Hal représente un atome d'halogène,
que l'on fait réagir sur un composé de formule (VII) : 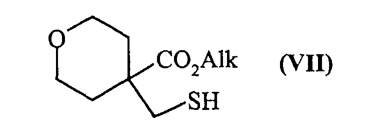 dans laquelle Alk représente un groupement alkyle (C₁-C₆) linéaire ou ramifié, pour conduire après réaction en milieu acide au composé de formule (VIII) : 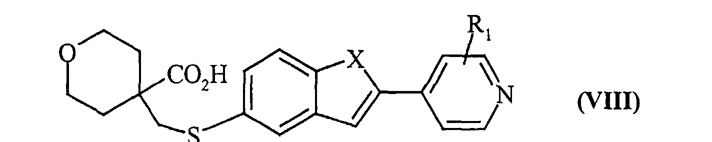 dans laquelle R₁ et X ont la même signification que dans la formule (I),
sur lequel on fait réagir un réactif d'oxydation,
pour conduire au composé de formule (IX) : 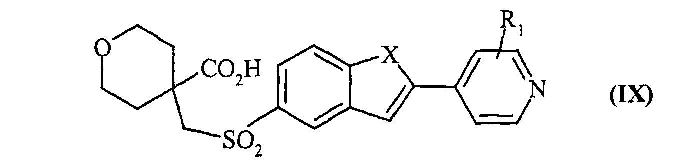 dans laquelle X et R₁ ont la même signification que dans la formule (I),
que l'on soumet à l'action d'une hydroxylamine-O-substituée, pour conduire après déprotection de la fonction hydroxamate au composé de formule (I/c), cas particulier des composés de formule (I) : 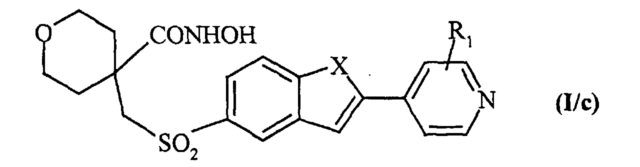 dans laquelle R₁ et X ont la même signification que dans la formule (I),
que l'on transforme éventuellement en N-oxydes correspondants,
que l'on purifie le cas échéant selon une technique classique de purification, dont on sépare éventuellement les isomères selon une technique classique de séparation et dont on transforme, si on le souhaite, en ses sels d'addition à un acide ou à une base pharmaceutiquement acceptable.

11. Compositions pharmaceutiques contenant comme principe actif au moins un composé selon l'une quelconque des revendications 1 à 8, seul ou en combinaison avec un ou plusieurs excipients ou véhicules inertes, non toxiques, pharmaceutiquement acceptables.

12. Compositions pharmaceutiques selon la revendication 11 contenant au moins un principe actif selon l'une quelconque des revendications 1 à 8 utiles comme inhibiteurs de métalloprotéases.

## Patentansprüche

1. Verbindungen der Formel (I): 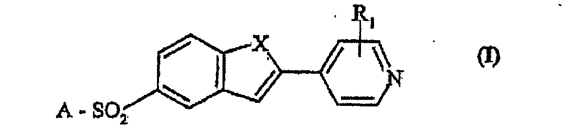 in der:
R₁ ein Wasserstoffatom, ein Halogenatom, eine geradkettige oder verzweigte (C₁-C₆)-Alkylgruppe oder eine geradkettige oder verzweigte (C₁-C₆)-Alkoxygruppe bedeutet,
X ein Sauerstoffatom, ein Schwefelatom oder eine Gruppe NR bedeutet, in der R ein Wasserstoffatom oder eine geradkettige oder verzweigte (C₁-C₆)-Alkylgruppe darstellt,
A eine der folgenden Gruppen bedeutet:
● 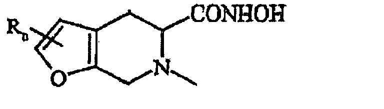 in der Rₐ ein Wasserstoffatom, ein Halogenatom, eine geradkettige oder verzweigte (C₁-C₆)-Alkylgruppe oder eine geradkettige oder verzweigte (C₁-C₆)-Alkoxygruppe darstellt,
● 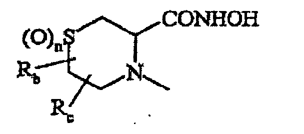 in der R_{b} und R_{c}, die gleichartig oder verschieden sind, ein Wasserstoffatom oder eine geradkettige oder verzweigte (C₁-C₆)-Alkylgruppe und n 0, 1 oder 2 bedeuten,
● oder 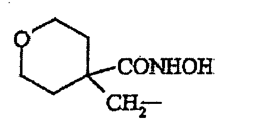
deren Isomere, N-Oxide sowie deren Additionssalze mit einer pharmazeutisch annehmbaren Säure oder Base.

2. Verbindungen der Formel (I) nach Anspruch 1, **dadurch gekennzeichnet, daß** A die folgende Gruppe bedeutet: 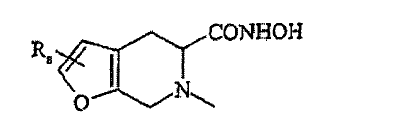 deren Isomere sowie deren Additionssalze mit einer pharmazeutisch annehmbaren Säure oder Base.

3. Verbindungen der Formel (I) nach Anspruch 1, **dadurch gekennzeichnet, daß** A die folgende Gruppe bedeutet: 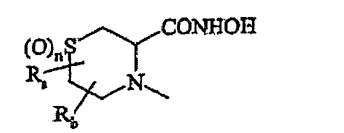 deren Isomere sowie deren Additionssalze mit einer pharmazeutisch annehmbaren Säure oder Base.

4. Verbindungen der Formel (I) nach Anspruch 1, **dadurch gekennzeichnet, daß** A die folgende Gruppe bedeutet: 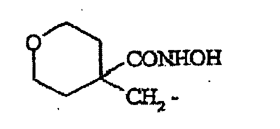 deren Isomere sowie deren Additionssalze mit einer pharmazeutisch annehmbaren Säure oder Base.

5. Verbindungen der Formel (I) nach Anspruch 3, **dadurch gekennzeichnet, daß** A die folgende Gruppe bedeutet: 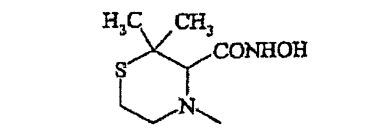 deren Isomere sowie deren Additionssalze mit einer pharmazeutisch annehmbaren Säure oder Base.

6. Verbindung der Formel (I) nach einem der Ansprüche 1 oder 2, nämlich N-Hydroxy-(5R)-6-{[2-(4-pyridinyl)-1-benzofuran-5-yl]-sulfonyl}-4,5,6,7-tetrahydro-[2,3-c]pyridin-5-carboxamid, dessen Isomere sowie dessen Additionssalze mit einer pharmazeutisch annehmbaren Säure oder Base.

7. Verbindung der Formel (I) nach einem der Ansprüche 1, 3 oder 5, nämlich N-Hydroxy-(3S)-2,2-dimethyl-4-{[2-(4-pyridinyl)-1-benzofuran-5-yl]-sulfonyl}-3-thiomorpholincarboxamid, dessen Isomere sowie dessen Additionssalze mit einer pharmazeutisch annehmbaren Säure oder Base.

8. Verbindung der Formel (I) nach einem der Ansprüche 1 oder 4, nämlich N-Hydroxy-4-{{[2-(4-pyridinyl)-1-benzofuran-5-yl]-sulfonyl}-methyl}-tetrahydro-2*H*-pyran-4-carboxamid, dessen Isomere sowie dessen Additionssalze mit einer pharmazeutisch annehmbaren Säure oder Base.

9. Verfahren zur Herstellung der Verbindungen der Formel (I) nach Anspruch 1, **dadurch gekennzeichnet, daß**, wenn die Verbindungen der Formel (I) jene sind, bei denen A eine der folgenden Gruppen bedeutet: 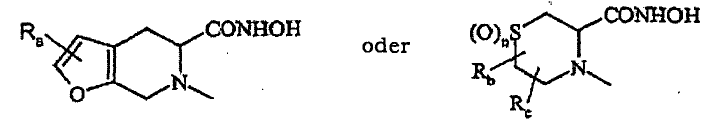 man als Ausgangsprodukt eine Verbindung der Formel (II) verwendet: 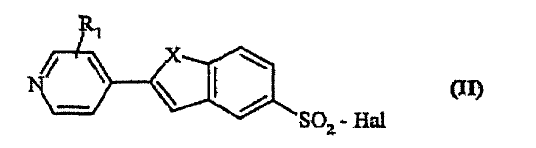 in der R₁ und X die bezüglich der Formel (I) angegebenen Bedeutungen besitzen und Hal für ein Halogenatom steht, welche man mit irgendeiner der Verbindungen (IIIa) oder (IIIb) in racemischer Form oder in Form des definierten Isomeren umsetzt: 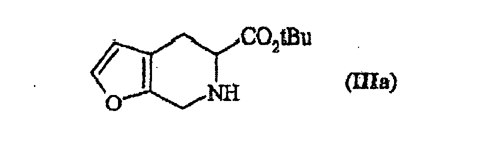 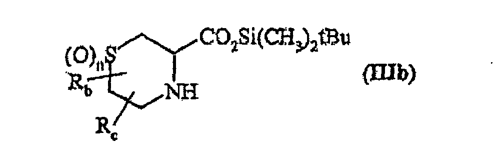 in denen Rb, Rc und n die bezüglich der Formel (I) angegebenen Bedeutungen besitzen, so daß man die Verbindungen der Formel (IVa) bzw. (IVb) erhält: 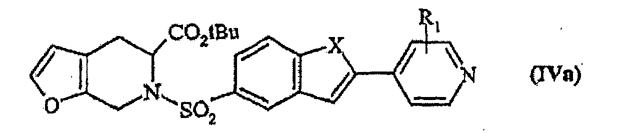 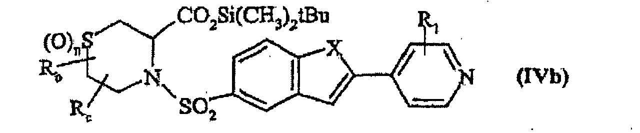 in denen X, R₁, Rb, Rc und n die bezüglich der Formel (I) angegebenen Bedeutungen besitzen,
von welchen man die Schutzgruppe abspaltet, so daß man die Verbindungen der Formel (Va) bzw. (Vb) erhält: 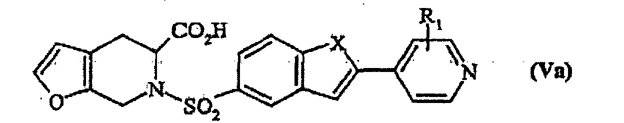 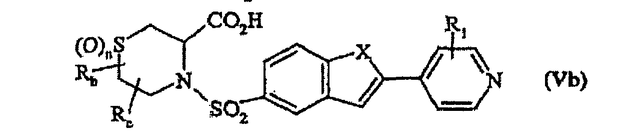 in denen X, R₁, Rb, Rc und n die bezüglich der Formel (I) angegebenen Bedeutungen besitzen,
welche man der Einwirkung eines O-substituierten Hydroxylamins unterwirft, so daß man nach der Abspaltung der Schutzgruppen der Hydroxamat-Funktion die Verbindungen der Formel (I/a) bzw. (I/b) erhält: 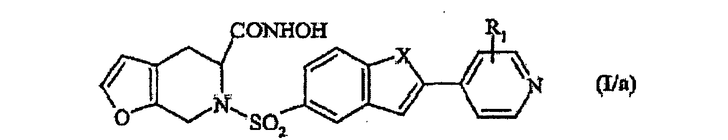 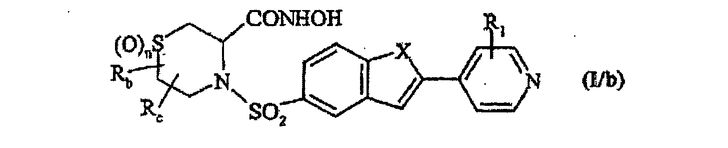 in denen X, R₁, Rb, Rc und n die bezüglich der Formel (I) angegebenen Bedeutungen besitzen,
welche Verbindungen der Formel (I/a) oder (I/b):
- man gegebenenfalls in die entsprechenden N-Oxide umwandelt,
- welche man erforderlichenfalls mit Hilfe einer klassischen Reinigungsmethode reinigt,
- welche man gegebenenfalls mit Hilfe einer klassischen Trennmethode in die Isomeren auftrennt und
- welche man gewünschtenfalls in ihre deren Additionssalze mit einer pharmazeutisch annehmbaren Säure oder Base überführt.

10. Verfahren zur Herstellung der Verbindungen der Formel (I) nach Anspruch 1, **dadurch gekennzeichnet, daß**, wenn die Verbindungen der Formel (I) solche sind, bei denen A die folgende Gruppe bedeutet: 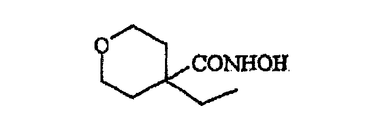 das Verfahren **dadurch gekennzeichnet ist, daß** man als Ausgangsprodukt eine Verbindung der Formel (VI) verwendet: 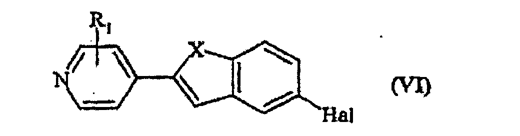 in der R₁ und X die bezüglich der Formel (I) angegebenen Bedeutungen besitzen und Hal für ein Halogenatom steht,
welche man mit einer Verbindung der Formel (VII) umsetzt: 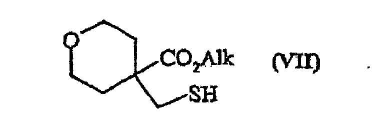 in der Alk eine geradkettige oder verzweigte (C₁-C₆)-Alkylgruppe darstellt, so daß man nach der Umsetzung in saurem Medium eine Verbindung der Formel (VIII) erhält: 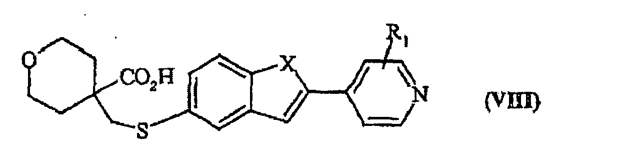 in der R₁ und X die bezüglich der Formel (I) angegebenen Bedeutungen besitzen, welche man mit einem Oxidationsmittel umsetzt,
zur Bildung der Verbindung der Formel (IX): 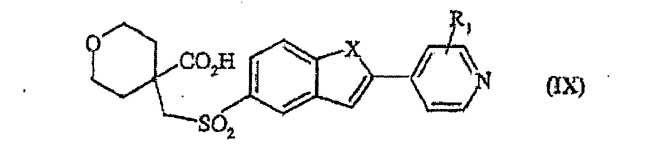 in der X und R₁ die bezüglich der Formel (I) angegebenen Bedeutungen besitzen, welche man der Einwirkung eines O-substituierten Hydroxylamins unterwirft, so daß man nach der Abspaltung der Schutzgruppe der Hydroxamat-Funktion die Verbindung der Formel (I/c) erhält, einem Sonderfall der Verbindungen der Formel (I): 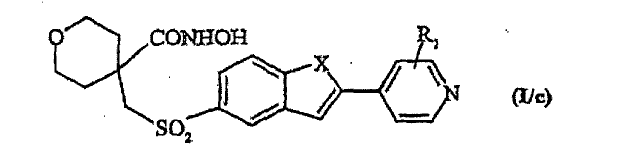 in der R₁ und X die bezüglich der Formel (I) angegebenen Bedeutungen besitzen, welche man gegebenenfalls in die entsprechenden N-Oxide umwandelt, welche man erforderlichenfalls mit Hilfe einer klassischen Reinigungsmethode reinigt, welche man gegebenenfalls mit Hilfe einer klassischen Trennmethode in ihre Isomeren trennt und welche man gewünschtenfalls in ihre Additionssalze mit einer pharmazeutisch annehmbaren Säure oder Base überführt.

11. Pharmazeutische Zubereitungen, enthaltend als Wirkstoff mindestens eine Verbindung nach einem der Ansprüche 1 bis 8, allein oder in Kombination mit einem oder mehreren inerten, nichttoxischen, pharmazeutisch annehmbaren Trägermaterialien oder Bindemitteln.

12. Pharmazeutische Zubereitungen nach Anspruch 11, enthaltend mindestens einen Wirkstoff nach einem der Ansprüche 1 bis 8 als Inhibitoren von Metalloproteasen.

## Claims

1. Compounds of formula (I) : 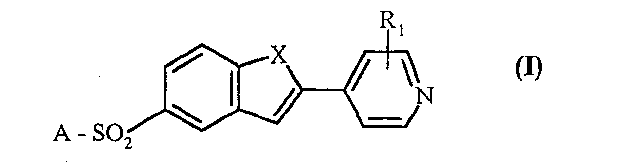 wherein :
R₁ represents a hydrogen atom, a halogen atom, a linear or branched (C₁-C₆)alkyl group or a linear or branched (C₁-C₆)alkoxy group,
X represents an oxygen atom, a sulphur atom or an NR group wherein R represents a hydrogen atom or a linear or branched (C₁-C₆)alkyl group,
A represents any one of the following groups :
● 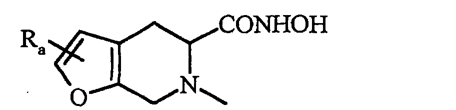 wherein Rₐ represents a hydrogen atom, a halogen atom, a linear or branched (C₁-C₆)alkyl group or a linear or branched (C₁-C₆)alkoxy group,
● 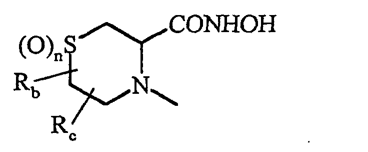 wherein R_{b} and R_{c}, which may be identical or different, represent a hydrogen atom or a linear or branched (C₁-C₆)alkyl group, and n is 0, 1 or 2,
or
● 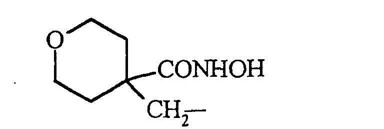
their isomers, N-oxides, and addition salts thereof with a pharmaceutically acceptable acid or base.

2. Compounds of formula (I) according to claim 1, **characterised in that** A represents the group: 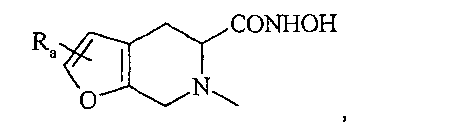 their isomers, and addition salts thereof with a pharmaceutically acceptable acid or base.

3. Compounds of formula (I) according to claim 1, **characterised in that** A represents the group : 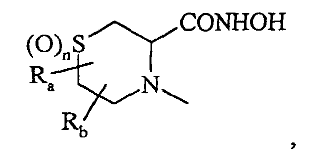 their isomers, and addition salts thereof with a pharmaceutically acceptable acid or base.

4. Compounds of formula (I) according to claim 1, **characterised in that** A represents the group : 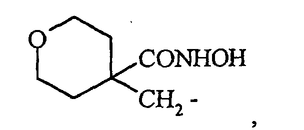 their isomers, and addition salts thereof with a pharmaceutically acceptable acid or base.

5. Compounds of formula (I) according to claim 3, **characterised in that** A represents the group : 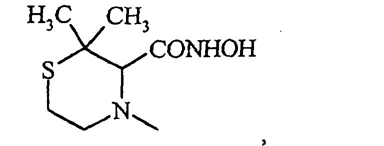 their isomers, and addition salts thereof with a pharmaceutically acceptable acid or base.

6. Compound of formula (I) according to either claim 1 or claim 2, which is N-hydroxy-(5R)-6-{[2-(4-pyridyl)-1-benzofuran-5-yl]sulphonyl}-4,5,6,7-tetrahydro[2,3-c]-pyridine-5-carboxamide, its isomers, and addition salts thereof with a pharmaceutically acceptable acid or base.

7. Compound of formula (I) according to any one of claims 1, 3 or 5, which is N-hydroxy-(3S)-2,2-dimethyl-4-{[2-(4-pyridyl)-1-benzofuran-5-yl]sulphonyl}-3-thiomorpholinecarboxamide, its isomers, and addition salts thereof with a pharmaceutically acceptable acid or base.

8. Compound of formula (I) according to either claim 1 or claim 4, which is N-hydroxy-4-{{[2-(4-pyridyl)-1-benzofuran-5-yl]sulphonyl}methyl}tetrahydro-2*H*-pyran-4-carboxamide, its isomers, and addition salts thereof with a pharmaceutically acceptable acid or base.

9. Process for the preparation of compounds of formula (I) according to claim 1, **characterised in that**, when the compounds of formula (I) are those wherein A represents any one of the groups : 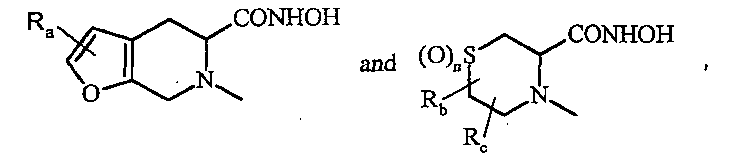 there is used as starting material a compound of formula (II) : 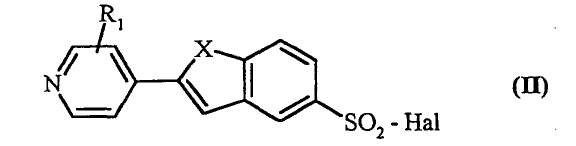 wherein R₁ and X are as defined for formula (I) and Hal represents a halogen atom, which is reacted with any one of compounds (IIIa) and (IIIb), in racemic form or in the form of a specific isomer: 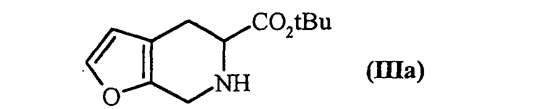 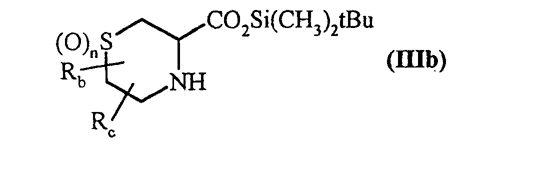 wherein Rb, Rc and n are as defined for formula (I),
to yield the compounds of formulae (IVa) and (IVb), respectively : 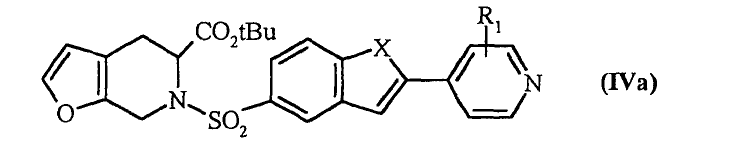 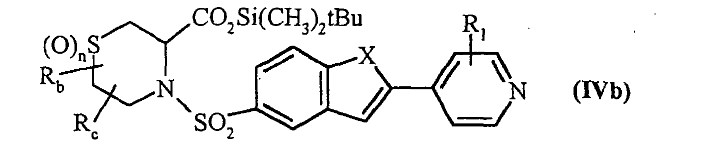 wherein X, R₁, Rb, Rc and n are as defined for formula (I),
which are deprotected to yield the compounds of formulae (Va) and (Vb), respectively, 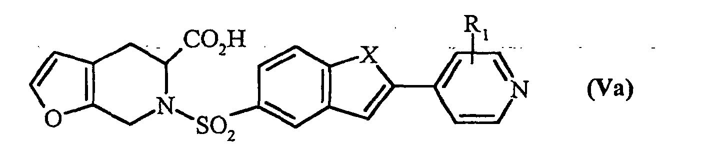 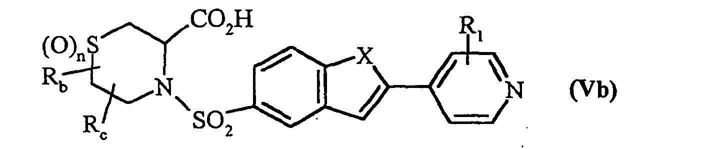 wherein X, R₁, Rb, Rc and n are as defined for formula (I),
which are subjected to the action of an O-substituted hydroxylamine, to yield, after deprotection of the hydroxamate function, the compounds of formulae (I/a) and (I/b), respectively : 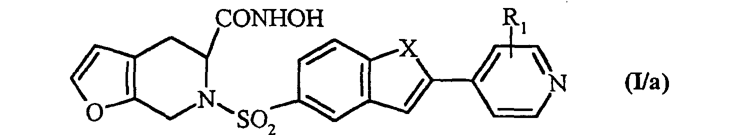 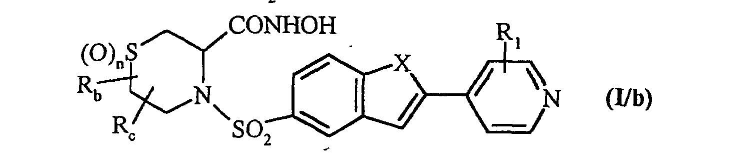 wherein X, R₁, Rb, Rc and n are as defined for formula (I),
which compounds of formulae (I/a) and (I/b) :
- are optionally converted to the corresponding N-oxides,
- are purified, if necessary, in accordance with a conventional purification technique,
- are separated, where appropriate, into their isomers in accordance with a conventional separation technique, and
- converted, if desired, into addition salts thereof with a pharmaceutically acceptable acid or base.

10. Process for the preparation of compounds of formula (I) according to claim 1, **characterised in that**, when the compounds of formula (I) are those wherein A represents the group: 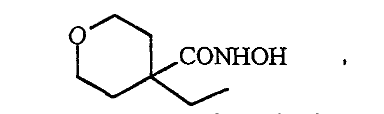 the process is **characterised in that** there is used as starting material a compound of formula (VI) : 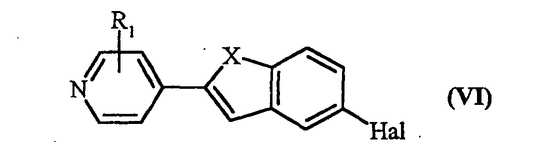 wherein R₁ and X are as defined for formula (I), and Hal represents a halogen atom,
which is reacted with a compound of formula (VII) : 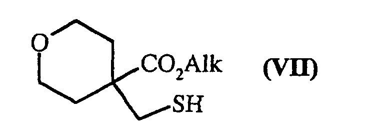 wherein Alk represents a linear or branched (C₁-C₆)alkyl group, to yield, after reaction in an acid medium, the compound of formula (VIII) : 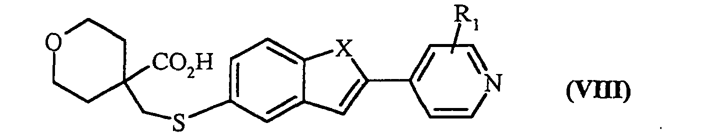 wherein R₁ and X are as defined for formula (I),
which is reacted with an oxidation reagent,
to yield the compound of formula (IX) : 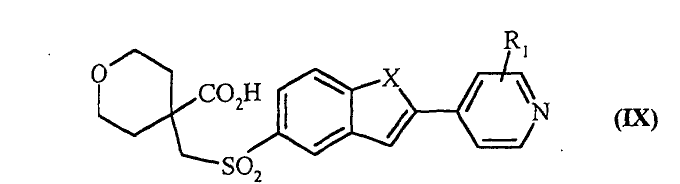 wherein X and R₁ are as defined for formula (I),
which is subjected to the action of an O-substituted hydroxylamine, to yield, after deprotection of the hydroxamate function, the compound of formula (I/c), a particular case of the compounds of formula (I) : 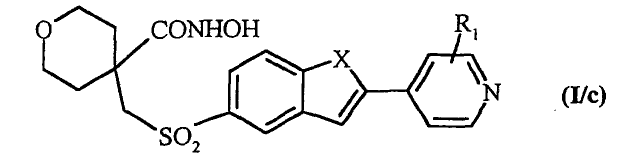 wherein R₁ and X are as defined for formula (I),
which is optionally converted to the corresponding N-oxide,
which is purified, if necessary, in accordance with a conventional purification technique, separated, where appropriate, into its isomers in accordance with a conventional separation technique, and converted, if desired, into addition salts thereof with a pharmaceutically acceptable acid or base.

11. Pharmaceutical compositions comprising as active ingredient at least one compound according to any one of claims 1 to 8, alone or in combination with one or more inert, nontoxic, pharmaceutically acceptable excipients or carriers.

12. Pharmaceutical compositions according to claim 11 comprising at least one active ingredient according to any one of claims 1 to 8 for use as metalloprotease inhibitors.
